# EUROPEAN PATENT SPECIFICATION

(11) **EP 0 451 184 B1**
(45) Date of publication and mention of the grant of the patent: **21.07.1993**
(21) Application number: 90901044.9
(22) Date of filing: 28.12.1989
(51) Int. Cl.: A61M 5/32, C08G 63/60

(54) **SINGLE-USE MEDICAL NEEDLE**
MEDIZINISCHE NADEL FÜR EINMALIGE VERWENDUNG
AIGUILLE MEDICALE A USAGE UNIQUE

(30) Priority: 29.12.1988 NL 8803204
(43) Date of publication of application: 16.10.1991
(73) Proprietor: NEDERLANDSE ORGANISATIE VOOR TOEGEPAST-NATUURWETENSCHAPPELIJK ONDERZOEK TNO, 2595 CL Den Haag (NL)
(72) Inventor: PEETERS, Johannes, Hendricus, Alphonsus, NL-3021 KE Rotterdam (NL); REIJSENBACH DE HAAN, Frederik, Karel, NL-5613 BA Eindhoven (NL)
(74) Representative: de Bruijn, Leendert C.
(86) International application number: NL8900099
(87) International publication number: WO9007348

(56) References cited:
- EP-A- 0 271 775
- EP-A- 0 284 726
- US-A- 2 512 568

## Description

The present invention relates to an needle suitable for medical and health care applications, which can easily be made unusable.

Needles for injection purposes and other applications, for example in health care, are typically made out of metal, since metals, such as stainless steel, have favourable properties for such applications, such as a high strength and relatively low price.

It is often desirable, however, that hypodermic needles and other needles cannot he used again after being used once,since any reuse implies the risk of infection. The need for single-use needles is especially pressing in order to prevent the spread of infection diseases, such as human immune system disorders (AIDS). One can only make sire that hypodermic and other needles are not being used again, by irreparably destroying the needle. With metal needles, such a destruction cannot be brought about easily. An additional disadvantage of metal needles, apart from the danger of infection, is the risk of injuries caused by their sharp points, even after partial destruction. Therefore, bending or breaking of the needle does not offer a solution. Making metal needles completely harmless requires complicated measures, such as casting a number of needles in a thermosetting resin. However, hypodermic needles and other needles for medical use are also often used in places of the world, where such facilities are not available.

Consequently there is a need for needles, for example for health care purposes, which share the advantages of metal needles (high bending strength, sharpness, sterilisability and the like), and which, in addition, can easily be made unsuitable for reuse.

A needle made of a polymeric material is disclosed in EP-A-0 271 775.

It has been surprisingly found now, that needles having the required properties can be made permanently unusable in a simple way, when the needle consists at least partly of a thermotropic liquid crystal polymer.

Because the rigid molecular chains of the liquid crystal polymer have been uniaxially directed during the forming process, the needle according to the invention has a high bending stiffness (self-reinforcement). The sharpness of the point required for injection and suture operations can also be easily provided. Further advantages of the needle according to the invention are its chemical and biological inertness and its satisfactory dimensioned stability. The material of the needle is resistent to elevated temperatures, allowing the needle to be simply sterilized.

The strength across the needle is lower than the longitudinal strength, but it is still high enough to avoid any risk of bending or breaking during use. By exerting a greater force in the transversal direction than occurring in normal use, the needle can be broken, cracked or crushed, readily rendering it unfit for further use. A very simple and effective way of making the needle unusable is the use of a flame (match, lighter, gas flame). This is preferably effected at a temperature around or above the melting temperature or the degradation temperature of the thermotropic liquid crystal polymer. As a result of the melting and/or degrading, the molecules loose their unidirectional orientation causing the material to deform, the needle point to loose its sharpness permanently and the hollow space in the needle to be lost.

The known liquid crystal polymers (LCP's) commercially available are suitable as a material for the needle according to the invention. In general, these are polymers, wherein so called mesogenic or "rigid" groups are present in the main chain or in the side chain. Such mesogenic groups are constituted of aromatic systems, such as phenylene naphthylene, biphenylene, pyrasinylene and benzoxazolylene groups and the like, linked by groups susceptible to conjugation, such as carbonyl, ester, amide, imine, azo groups and the like. In the preferably used polymers, the mesogenic groups are situated in the main chain. Suitable LCP's have liquid crystal behaviour in the melt (thermotropic) or in solution (lyotropic). Examples of thermotropic LCP's are aromatic polyesters and examples of lyotropic LCP's are aromatic polyamides (aramides). Thermotropic LCP's are preferred because they can be processed more conveniently (through the melt). The stiffness and melt behaviour of the LCP's can be varied in a known way by changing the ratio between mesogenic (rigid) groups and flexible groups, such as alkylene groups, or by incorporating angle forming groups such as meta substituted aromatic groups and the like. The polyesters can be based for example on terephthalic acid, aromatic diols and hydroxy substituted carboxylic acids. Examples of commercially available thermotropic LCP's are Vectra (Celanese-Hoechst), Ultrax (BASF), Xydar (Dartco), X7G (Idimitsu) and Victrex (ICI). Satisfactory results are obtained using Vectra, as this polyester exhibits less fibrillation.

Other suitable thermotropic LCP's are described in European Patent Application 284.726. Lyotropic LCP's such as Kevlar (DuPont) and Twaron (Akzo) can be processed in solution.

If a particularly high stiffness is required for a special application, the LCP can be blended with reinforcement fibres, such as glass fibres, carbon fibres etc. If on the other hand high flexibility is desired, such as for infusion purposes, the LCP can be mixed with a thermoplast polymer. Coating with a biocompatible polymer is another possibility contemplated according to the invention.

The needle according to the invention, which can be solid or hollow, can be shaped as desired in any convenient way known in itself, whereby the required uniaxial orientation is obtained (elongational flow). If necessary, a final stretching can be performed for obtaining an even stronger orientation. A solid needle can be shaped for example by injection moulding. Solid needles are suitable, for example for surgical applications (sutures and the like) and can have any convenient shape (straight, curved etc.) and size: the diameter can be for instance 0.2 to 3 mm, depending on the length. The needle according to the invention is preferably hollow and therefore suitable for hypodermic injection of liquids. The hollow needle can also be shaped by injection moulding, but in this case extrusion or fibre spinning offers processing advantages. The hollow needle can be combined with conventional needle holders and liquid containers to provide syringes, infusions etc. The combined needle, needle holder and possibly the liquid container can be manufactured as a single integrated product by means of injection moulding. The hollow needle can have any desired size. The diameter of the needle is for example 0.2-3 mm and the wall thickness can be for example 0.05-0.5 mm depending on the length. The needle can be bevelled in a known way, for example by grinding or cutting. The quantity of material needed for the needle according to the invention is very small; for example, for a hypodermic needle it is usually considerably less than 100 mg; the cost price per disposable needle is thus very low, so that the use of the somewhat expensive LCP's does not raise any economical drawbacks.

## Claims

1. Needle, suitable for medical and health care use, which can easily be made unusable, characterized in that is consists at least partly of a thermotropic liquid crystal polymer.

2. Needle according to claim 1, characterized in that it is hollow and suitable for injection of liquids.

3. Needle according to claim 1 or 2, characterized in that the liquid crystal polymer comprises an aromatic polyester.

4. Process of making a needle according to any of claims 1-3 unsusable, characterized in that it is exposed to a temperature around or above the melting or degrading temperature of the liquid crystal polymer for a short period of time.

## Patentansprüche

1. Nadel, die sich für medizinische und prophylaktische Zwecke eignet und leicht unbrauchbar gemacht werden kann, dadurch **gekennzeichnet,** daß sie zumindest teilweise aus einem thermotrophen Flüssigkristall-Polymer besteht.

2. Nadel nach Anspruch 1, dadurch **gekennzeichnet,** daß sie hohl ist und sich zur Injektion von Flüssigkeiten eignet.

3. Nadel nach Anspruch 1 oder 2, dadurch **gekennzeichnet,** daß das Flüssigkristall-Polymer einen aromatischen Polyester umfaßt.

4. Verfahren zum Unbrauchbarmachen einer Nadel nach einem der Ansprüche 1 bis 3, dadurch **gekennzeichnet,** daß man sie einer Temperatur im Bereich oder oberhalb der Schmelz- oder Zersetzungstemperatur des Flüssigkristall-Polymers für kurze Zeit aussetzt.

## Revendications

1. Aiguille appropriée pour les traitements médicaux et les soins, pouvant être rendue facilement inutilisable, caractérisée en ce qu'elle comprend, au moins en partie, un polymère cristallin liquide thermotropique.

2. Aiguille selon la revendication 1, caractérisée en ce qu'elle est creuse et qu'elle convient pour l'injection de liquides.

3. Aiguille selon la revendication 1 ou 2, caractérisée en ce que le polymère cristallin liquide comprend un polyester aromatique.

4. Procédé pour rendre inutilisable une aiguille à usage unique selon l'une quelconque des revendications 1 à 3, caractérisée en ce qu'elle est exposée à une température voisine ou supérieure à la température de fusion ou de dégradation du polymère cristallin liquide pendant une courte période de temps.
